# EUROPEAN PATENT SPECIFICATION

(11) **EP 4 126 162 B1**
(45) Date of publication and mention of the grant of the patent: **11.03.2026**
(21) Application number: 21718737.6
(22) Date of filing: 23.03.2021
(51) Int. Cl.: A61M 25/00, A61M 25/06, A61B 5/15, A61M 39/22, A61M 39/00

(54) **CATHETER ASSEMBLY WITH A SLIDABLE SEPTUM AND RELATED SYSTEMS**
KATHETERANORDNUNG MIT VERSCHIEBBAREM SEPTUM UND ZUGEHÖRIGE SYSTEME
ENSEMBLE CATHÉTER À SEPTUM COULISSANT ET SYSTÈMES ASSOCIÉS

(30) Priority: 31.03.2020 US 202063002972 P; 22.03.2021 US 202117208975
(43) Date of publication of application: 08.02.2023
(73) Proprietor: Becton, Dickinson and Company, Franklin Lakes, NJ 07417 (US)
(72) Inventor: NADU, Jithendra Kumar Sathyanarayana, 731682 Woodlands (SG)
(74) Representative: dompatent
(86) International application number: PCT/US2021/023644
(87) International publication number: WO 2021/202167

(56) References cited:
- WO-A1-2018/217781
- US-A- 5 098 405
- US-A1- 2007 221 275
- US-A1- 2013 090 607
- US-A1- 2015 018 802

## Description

### BACKGROUND

A catheter is commonly used to infuse fluids into vasculature of a patient. For example, the catheter may be used for infusing normal saline solution, various medicaments, or total parenteral nutrition. The catheter may also be used for withdrawing blood from the patient.

In either case, an over-the-needle peripheral intravenous ("IV") catheter may be mounted over an introducer needle, which may include a sharp distal tip. The catheter and the introducer needle may be assembled so that the distal tip of the introducer needle extends beyond the distal tip of the catheter, with the bevel of the needle facing up away from skin of the patient. The catheter and introducer needle are generally inserted at a shallow angle through the skin into vasculature of the patient.

Before introducing the catheter into the vasculature of the patient, however, air present in the catheter system is removed to prevent air from entering the patient's bloodstream. This "priming" process may be performed by flushing the catheter system with saline, for example. Then, to verify proper placement of the introducer needle and/or the catheter in the blood vessel, a clinician generally confirms that there is "flashback" of blood in a flashback chamber of the catheter assembly. Once placement of the needle has been confirmed, the clinician may temporarily occlude flow in the vasculature and remove the needle, leaving the catheter in place for future blood withdrawal or fluid infusion.

Unfortunately, the presence of priming solution in the catheter system tends to compromise continuous blood flow through the catheter, thereby impeding the ability to obtain a quick and clear indication of flashback. This may lead to confusion or uncertainty regarding whether the catheter has been properly inserted into the patient's vasculature. Proper catheter insertion may be delayed as a result, causing prolonged exposure of the catheter to the priming solution. This, in turn, may cause the catheter material to soften, thereby reducing catheter tip stiffness and column strength. During insertion, the catheter material may get stuck outside of the vein, causing catheter peel back and possible vein damage.

The subject matter claimed herein is not limited to embodiments that solve any disadvantages or that operate only in environments such as those described above. Rather, this background is only provided to illustrate one example technology area where some implementations described herein may be practiced.

US 2015/0018802 A1 discloses a catheterization apparatus.

WO 2018/217781 A1 discloses a catheter hub with an injections port and a valve.

US 2007/0221275 A1 discloses a shuttle valve.

US 5,098,405 B discloses an apparatus for a side port catheter adapter with a one piece integral combination valve.

US 2013/0090607 A1 discloses an intravenous catheter with a duckbill valve.

### SUMMARY

The invention is defined in independent claims 1 and 4.

Further aspects are defined in respective dependent claims 2,3 and 5-7.

The present disclosure generally relates to an intravenous catheter assembly, as well as to related devices, systems, and methods (not claimed).

In some embodiments, a catheter assembly may increase a likelihood of obtaining a quick and clear indication of flashback to confirm proper catheter placement. In some embodiments, the catheter assembly may thereby avoid delays in catheter insertion resulting from clinician confusion or uncertainty regarding proper catheter placement.

In some embodiments, the catheter assembly may also prevent prolonged exposure of a catheter to priming solution. Some embodiments of the catheter assembly may thus reduce a risk of one or more of the following: a catheter material softening, reduced catheter tip stiffness, and reduced column strength. Reducing prolonged exposure of the catheter to priming solution may also reduce a risk of catheter peel back and/or resulting vein damage.

The catheter assemblyincludes a catheter adapter, the catheter, and a septum. The catheter adapter includes a distal end, a proximal end, and a lumen extending therethrough. A side port is disposed between the distal end and the proximal end of the catheter adapter, and a catheter may extend from the distal end. The septum is disposed within the lumen at a distal position aligned with the side port. In the distal position, the septum provides a seal between the side port and the catheter.

The septum is configured to slide from the distal position to a proximal position proximal to the side port. In the proximal position, the side port is in fluid communication with the catheter. In this manner, the catheter assembly may isolate a blood fluid pathway from a priming solution fluid pathway and also facilitate selective retraction of the needle septum to later join the blood fluid pathway and the priming solution fluid pathway.

A puller element is disposed within the lumen of the catheter adapter. The puller element includes a distal end coupled to the septum and a proximal end configured to couple to a blood collection set. The catheter adapter includes a **slot,** which includes a stop at a proximal end thereof. The puller element includes an extension extending through the slot and configured to contact the stop when the septum slides from the distal position to the proximal position. In some embodiments, the extension may be coupled to a push tab.

In some embodiments, the catheter adapter may include an air vent. In some embodiments, an outer surface of the septum may include an annular groove. In some embodiments, in response to the septum being in the distal position, the side port may be in fluid communication with the annular groove and the air vent. In some embodiments, a stop may be disposed within the lumen of the catheter adapter. In some embodiments, the stop may be formed by the air vent.

A catheter system in an embodiment includes the catheter assembly and a needle assembly. The needle assembly includes a needle hub coupled to the proximal end of the catheter adapter. The needles assembly includes an introducer needle, which includes a sharp distal tip, a proximal end, and a needle feature, such as a bump, disposed between the sharp distal tip and the proximal end. The proximal end of the introducer needle is secured within the needle hub.

The introducer needle is withdrawn from the catheter adapter in a proximal direction in a first amount. In response, the needle feature is configured to contact the septum and slide the septum from the distal position to a proximal position such that the side port is in fluid communication with the catheter and the stop may contact the septum. In some embodiments, withdrawal of the introducer needle in the proximal direction more than the first amount causes the needle feature to move proximally with respect to the septum.

In some embodiments, the outer surface of the septum may include an annular groove. In response to the septum being in the distal position, the side port may be in fluid communication with the annular groove and the air vent.

In some embodiments, a method (not part of the claimed invention and left for illustrative purposes) may include priming the catheter system with a priming solution. In some embodiments, the air vent may extend through a wall of the catheter adapter.

In some embodiments, a needle assembly may include the introducer needle, which may include the sharp distal tip, the proximal end, and a notch. After priming the catheter system, the introducer needle and the catheter may be inserted into vasculature. In response, blood may flow into the sharp distal tip of the introducer needle, through the notch into a space between the introducer needle and the catheter, and proximate the septum. In some embodiments, the blood may be isolated such that it is not in fluid communication with the priming solution.

In some embodiments, the needle assembly may include a flash chamber. In some embodiments, blood may flow through the introducer needle to the flash chamber in response to the introducer needle being inserted into the vasculature.

In some embodiments, the introducer needle may include the needle feature, such as the bump, disposed between the sharp distal tip and the proximal end of the introducer needle. In some embodiments, after inserting the introducer needle and the catheter into the vasculature, the introducer needle may be withdrawn in the proximal direction in the first amount such that the needle feature contacts the septum and slides the septum from the distal position to the proximal position.

In some embodiments, after withdrawing the introducer needle in the proximal direction the first amount, the introducer needle may be withdrawn in the proximal direction more than the first amount. This may cause the needle feature to move proximally with respect to the septum. In some embodiments, in response to withdrawing the introducer needle in the proximal direction the first amount, the needle feature may contact the septum and slide the septum from the distal position to the proximal position.

In some embodiments, the catheter assembly may further include the puller element disposed within the lumen. In some embodiments, the puller element may include the distal end coupled to the septum and the proximal end configured to couple to the blood collection set. In some embodiments, the catheter adapter may further include the slot. In some embodiments, a proximal end of the slot may include the stop. In some embodiments, the puller element may further include the extension extending through the slot and configured to contact the stop in response to the septum sliding from the distal position to the proximal position.

Some embodiments of the method may include removing the needle assembly from the catheter system. In some embodiments, a distal end of a housing of the blood collection set may be coupled to the proximal end of the puller element. In some embodiments, the housing may include one or more of the following: a distal end, a proximal end, a slot, and a cannula, which may include a wing extending through the slot. In some embodiments, the cannula hub may be slidable with respect to the housing between a retracted position and an advanced position, and a cannula may extend distally from the cannula hub. In some embodiments, the blood collection set may also include an extension tube, which may include a distal end coupled to the proximal end of the cannula hub. In some embodiments, a blood collection device may be coupled to the proximal end of the extension tube. In some embodiments, the blood collection device may include a syringe, an evacuated blood collection tube, a small sample collection device, or another suitable blood collection device.

In some embodiments, after coupling the distal end of the housing of the blood collection set to the proximal end of the puller element, the method may include gripping the wing to slide the cannula hub from the retracted position to the advanced position. In some embodiments, the cannula may be disposed within the housing in response to the cannula hub being in the retracted position. In some embodiments, in response to the cannula hub being in the advanced position, the cannula may extend through the distal end of the housing, the puller element, and the septum.

In some embodiments, after gripping the wing to slide the cannula hub from the retracted position to the advanced position, the method may include collecting a blood sample within the blood collection set. In some embodiments, the method may further include, after collecting the blood sample within the blood collection set, gripping the wing to slide the cannula hub from the advanced position to the retracted position.

In some embodiments, after collecting the blood sample within the blood collection set, the housing may be moved proximally until the extension contacts the stop. In some embodiments, in response to moving the housing proximally, the septum may slide from the distal position to the proximal position. In some embodiments, the housing may be uncoupled from the puller element in response to the extension contacting the stop.

In some embodiments, the extension may be coupled to a tab. In these embodiments, moving the housing proximally until the extension contacts the stop may include gripping the housing and the tab.

It is to be understood that both the foregoing general description and the following detailed description are examples and explanatory and are not restrictive of the invention, as claimed. It should be understood that the various embodiments are not limited to the arrangements and instrumentality shown in the drawings. It should also be understood that the embodiments may be combined, or that other embodiments may be utilized and that structural changes, unless so claimed, may be made without departing from the scope of the various embodiments of the present invention. The following detailed description is, therefore, not to be taken in a limiting sense.

### BRIEF DESCRIPTION OF THE SEVERAL VIEWS OF THE DRAWINGS

Example embodiments will be described and explained with additional specificity and detail through the use of the accompanying drawings in which:
Figure 1 is an upper perspective view of an example catheter system, in accordance with some embodiments;
Figure 2 is an exploded view of the catheter system of Figure 1, in accordance with some embodiments;
Figure 3 is an upper perspective view of another example catheter system, illustrating a needle having a needle feature and a septum in a distal position aligned with a side port, in accordance with some embodiments;
Figure 4 is a cross-sectional view of the catheter system of Figure 3, illustrating the septum in the distal position, in accordance with some embodiments;
Figure 5 is an upper perspective view of an example catheter assembly of the catheter system of Figure 3, illustrating a septum in a proximal position proximal to a side port, in accordance with some embodiments;
Figure 6 is a cross-sectional view of the catheter assembly of Figure 5, illustrating the septum in the proximal position, in accordance with some embodiments;
Figure 7 is an upper perspective view of an example catheter assembly of the catheter system of Figure 1, illustrating a puller element and a septum in a distal position aligned with a side port, in accordance with some embodiments;
Figure 8 is a cross-sectional view of the catheter assembly of Figure 7, illustrating an example blood collection set coupled to the puller element and the septum in the distal position, in accordance with some embodiments;
Figure 9 is an upper perspective view of the catheter assembly of Figure 7, illustrating the septum positioned in a proximal position proximal to the side port according to some embodiments;
Figure 10 is a cross-sectional view of the catheter assembly of Figure 9, illustrating the septum in the proximal position, in accordance with some embodiments;
Figure 11A is an upper perspective view of an example catheter system, illustrating a priming step in accordance with some embodiments;
Figure 11B is an upper perspective view of the catheter system of Figure 11A, illustrating a flashback step, in accordance with some embodiments;
Figure 11C is an upper perspective view of the catheter system of Figure 11A, illustrating engagement of a blood collection set, in accordance with some embodiments;
Figure 11D an upper perspective view of the catheter assembly of the catheter system of Figure 11A, illustrating a blood collection step, in accordance with some embodiments;
Figure 11E is a cross-sectional view of the catheter assembly of the catheter system of Figure 11A and blood collection step of Figure 11D, in accordance with some embodiments;
Figure 11F is an upper perspective view of the catheter assembly of the catheter system of Figure 11A, illustrating an infusion step, in accordance with some embodiments; and
Figure 11G is an upper perspective view of an example blood collection set, according to some embodiments.

### DESCRIPTION OF EMBODIMENTS

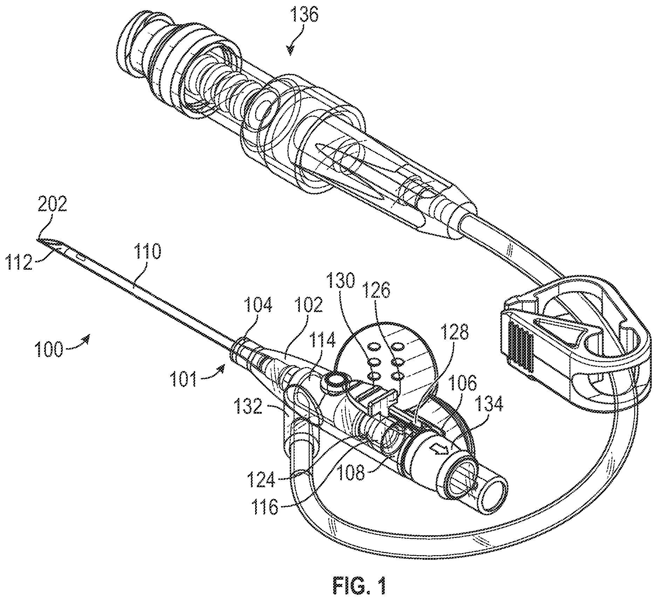

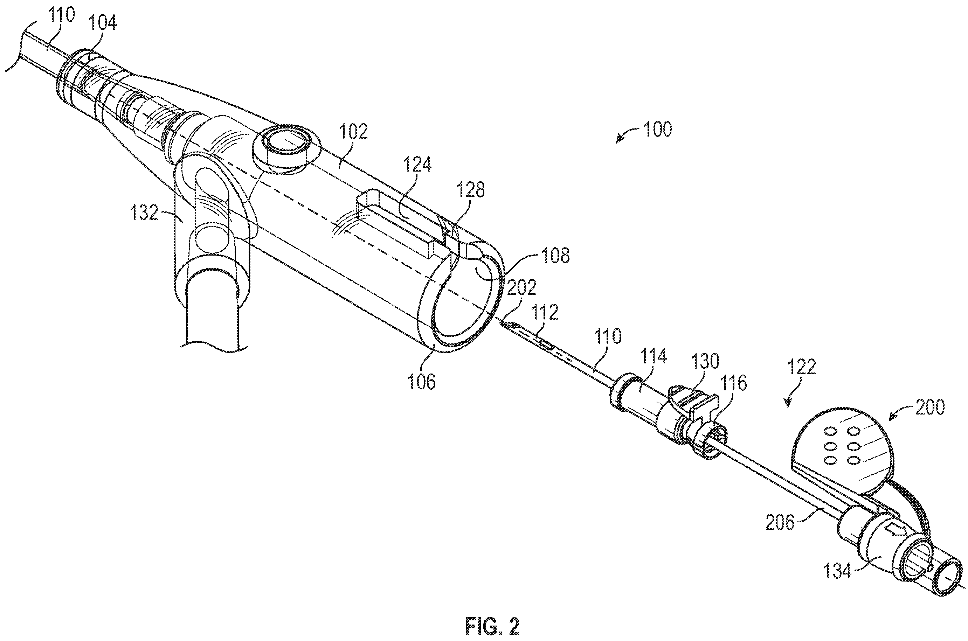

Referring now to Figures 1 and 2, in some embodiments, to introduce a catheter 110 into a patient's vasculature, a catheter 110 may be mounted over a hollow-bore introducer needle 112, which may include a sharp distal tip 202. In some embodiments, an inner surface of the catheter 110 may tightly engage the outer surface of the introducer needle 112 to prevent catheter 110 peel back and to facilitate insertion of the catheter 110 into a blood vessel or vein. In some embodiments, the sharp distal tip 202 of the introducer needle 112 may extend beyond the distal tip of the catheter 110 to facilitate insertion of the catheter 110 at a shallow angle through the patient's skin and into a vein.

In some embodiments, a catheter system 100 may include a catheter assembly 101. In some embodiments, a catheter assembly 101 may include a catheter adapter 102, which may include a lumen 108 extending longitudinally between a distal end 104 and a proximal end 106 thereof. In some embodiments, the lumen 108 may have an inner diameter at least slightly larger than an outer diameter of a catheter 110, which may be over-the-needle. In some embodiments, the catheter 110 may be coupled to the distal end 104 of the catheter adapter 102. In some embodiments, the catheter 110 may be secured and mechanically sealed to the catheter adapter 102 by way of, for example, a threaded connection, a press fit, a wedge, or by any other means known to those in the art.

In some embodiments, the inner diameter of the lumen 108 may increase between the distal end 104 and the proximal end 106 of the catheter adapter 102. In some embodiments, an inside diameter of the catheter adapter 102 at the distal end 104 may substantially match an outside diameter of an associated catheter 110 to tightly engage an introducer needle 112 therein. This may prevent peel back of the catheter 110 during insertion of the catheter 110 into a blood vessel. In some embodiments, the proximal end 106 of the catheter adapter 102, on the other hand, may include a substantially larger inside diameter to receive and/or engage a blood collection set or other peripheral device.

In some embodiments, a side port 132 may extend from the catheter adapter 102 and communicate with the lumen 108. In this manner, the side port 132 may provide an independent fluid pathway between the catheter 110 and an infusion set or other infusion device coupled to the side port 132. In some embodiments, the infusion set may be coupled to a needleless access connector 136 or directly to an adapter disposed at a proximal end of an extension tube extending from the side port 132.

In some embodiments, the side port 132 may be used to provide an independent fluid pathway for dispensing a priming fluid or solution, such as saline or the drug intended for infusion, to prime the catheter system 100. Similarly, in some embodiments, the side port 132 may provide an independent fluid pathway to infuse fluids into a patient's vasculature. Fluids may include, for example, saline solution, various medicaments, total parenteral nutrition, and the like.

In some embodiments, a septum 114 may be disposed within the lumen 108 of the catheter adapter 102. In some embodiments, the septum 114 may include a substantially resilient and fluid impervious material to facilitate creating an effective fluid seal between the side port 132 and the lumen 108. In some embodiments, the septum 114 may include a material, such as silicone, foam, rubber, a composite, or the like. In some embodiments, the material may be configured to expand or otherwise substantially occupy the interior space or volume of the lumen 108. In some embodiments, the septum 114 and/or the lumen 108 may include a coating or lubricant to facilitate the ability of the septum 114 to slide within the lumen 108 and/or seal the fluid pathway between the lumen 108 and the side port 132.

Further, in some embodiments, the septum 114 may include dimensions sufficient to selectively occlude and seal a fluid pathway between the side port 132 and the lumen 108. In some embodiments, the septum 114 may include a substantially circular cross-section having a diameter substantially matching a diameter of the lumen 108 of the catheter adapter 102. The septum 114 may further include a length and height sufficient to occlude a distal opening 138 of the side port 132. Of course, the septum 114 may include any shape and/or dimensions to seal a fluid pathway between the side port 132 and the lumen 108.

As discussed in more detail below, in some embodiments, the septum 114 may be configured to slide and/or otherwise move within the lumen 108 between a distal position and a proximal position. In some embodiments, in the distal position, the septum 114 may be substantially aligned with the side port 132 such that the septum 114 provides a seal between the side port 132 and the catheter 110. In this manner, a fluid pathway between the distal end 104 and the proximal end 106 of the catheter adapter 102 may be isolated to prevent contamination from current or residual fluid in the side port 132. In the proximal position, on the other hand, the septum 114 may create a pathway between the side port 132 and the catheter adapter 102 to facilitate the side port 132 to be in fluid communication with the catheter 110.

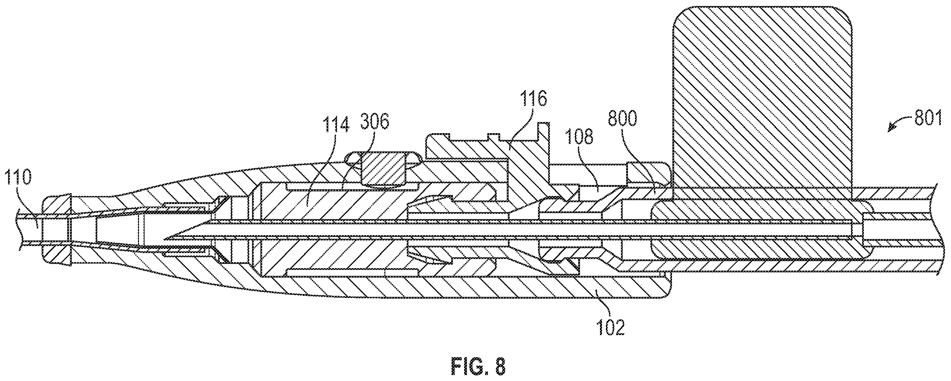

In some embodiments, manipulating a position of the septum 114 in this manner may prevent one fluid from contaminating or diluting another. In some embodiments, priming solution may be dispensed via the side port 132 to prime the catheter system 100. In some embodiments, the fluid pathway used to dispense the priming solution may be sealed with respect to the lumen 108 of the catheter adapter 102. This may enable pure or undiluted blood to be drawn from a cannula of a blood collection set (see, for example, the blood collection set 801 of Figure 8), which may be coupled to a proximal end 106 of the catheter adapter 102. In some embodiments, after collecting the blood sample, the septum 114 may be moved from the distal position to the proximal position within the lumen 108 to create a fluid pathway through the catheter 110 and the side port 132 for fluids intended for infusion. In this manner, undiluted blood may be drawn from the catheter 110, which may be a same catheter used for priming and infusion, with little or no risk of cross-contamination between any of the various fluids. In some embodiments, the catheter 110 may include a peripheral intravenous catheter ("PIVC"), a midline catheter, or a peripherally-inserted central catheter ("PICC").

Of course, one skilled in the art will recognize that embodiments of the present disclosure are not limited to any particular fluids and/or number or orientation of fluid pathways, as all fluids and any number and/or orientations of fluid pathways are contemplated herein. For example, a length of the septum 114 may be extended or the dimensions of the septum 114 may be modified to enable more than one side port 132 to be sealed simultaneously. In other embodiments, the side port 132 orientation may be varied to create any number of fluid pathways. For example, two or more side ports 132 may be disposed on opposite sides of the catheter adapter 102 such that translating the septum 114 within the lumen 108 may expose more than one side port 132 at a time.

In some embodiments, a puller element 116 may be disposed within the lumen 108 such that a distal end 118 of the puller element 116 is positioned substantially adjacent to a proximal end of the septum 114. In some embodiments, a proximal end 120 of the puller element 116 may be configured to couple to, for example, a needle hub 134 and/or the blood collection set. In other embodiments, the distal end 118 of the puller element 116 may be integrated with or coupled to the proximal end of the septum 114. In some embodiments, the puller element 116 and the septum 114 may be monolithically formed as a single unit. In some embodiments, the distal end 118 of the puller element 116 may be positioned substantially adjacent to the proximal end of the septum 114.

In some embodiments, the catheter adapter 102 may include a slot 124 to receive at least a portion of the puller element 116. For example, in some embodiments, the puller element 116 may include an extension 126 extending through the slot 124. In some embodiments, a push tab 130 may be coupled to an end of the extension 126 to enable manual translation of the puller element 116 through a wall of the catheter adapter 102.

In some embodiments, a proximal end of the slot 124 may include a stop 128, such as a narrowing, edge, ridge, or any other suitable feature that is integrated with or coupled to the slot 124. In some embodiments, the stop 128 may contact the extension 126 when the septum 114 is moved from the distal position to the proximal position.

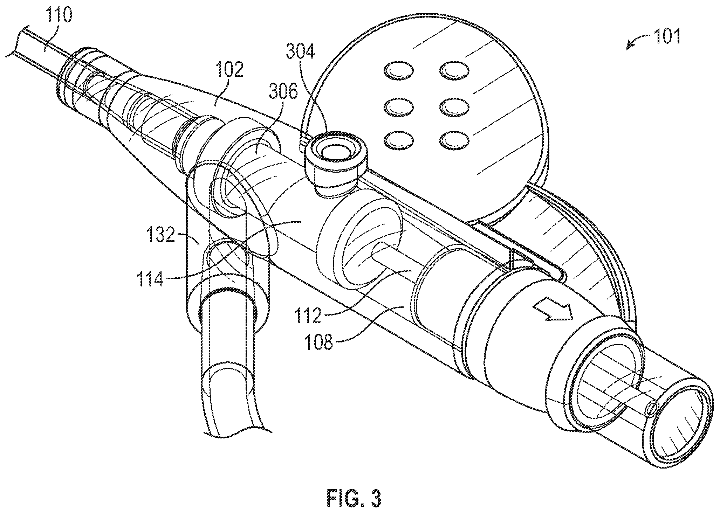

Referring now to Figures 3 - 6, some embodiments of a catheter system 100 may include a needle assembly 200, which may include a needle hub 134 coupled to the proximal end 106 of the catheter adapter 102. In some embodiments, a proximal end 206 of an introducer needle 112 may be secured within the needle hub 134 by, for example, a threaded connection or a press fit. Alternatively, in some embodiments, the introducer needle 112 may be secured within the needle hub 134 by any means known to those in the art. In some embodiments, the introducer needle 112 may include a sharp distal tip 202 and a needle feature 204 disposed between the sharp distal tip 202 and the proximal end 206. In some embodiments, at least a portion of a length of the introducer needle 112 may extend through the septum 114 and/or puller element 116.

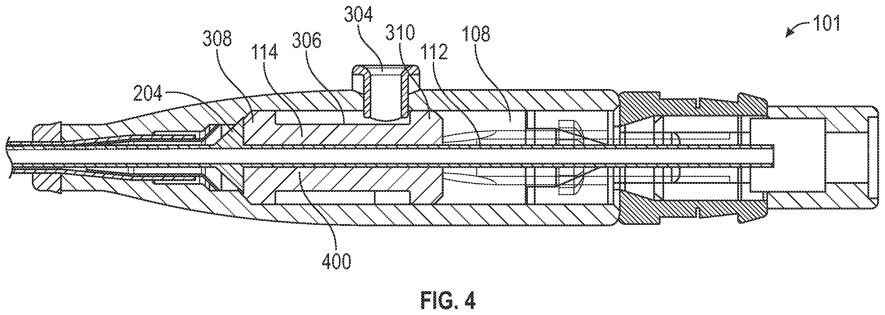

As illustrated in Figures 3 and 4, in some embodiments, the sharp distal tip 202 of the introducer needle 112 may be disposed distal to the septum 114 and/or puller element 116. In some embodiments, the septum 114 may include a slit, through which the introducer needle 112 may extend. In some embodiments, the needle feature 204, such as a bump, crimp, or another suitable feature, may be disposed distal to the septum 114.

In certain embodiments, an air vent 304 may be integrated with or coupled to an aperture in the outer surface of the catheter adapter 102 to facilitate air to flow between the lumen 108 of the catheter adapter 102 and an external environment. Some embodiments of an air vent 304 may include a hydrophobic membrane to avoid any interference with priming fluid or any other fluid or solution. In some embodiments, the hydrophobic membrane may be permeable to air but not priming fluid or any other fluid or solution.

In some embodiments, an outer surface of the septum 114 may include an annular groove 306 disposed between its distal end and its proximal end. This design may relieve frictional force when the septum 114 is translated between the distal position and the proximal position. In some embodiments, the annular groove 306 may also create a distal edge 308 at the distal end of the septum 114 and proximal edge 310 at the proximal end of the septum 114. In some embodiments, when the septum 114 is in the distal position, the side port 132 may be in fluid communication with the annular groove 306 and the air vent 304. In certain embodiments, the air vent 304 may form a stop to contact the proximal edge 310 of the septum 114 within the lumen 108. In some embodiments, the stop may secure the position of the septum 114 with respect thereto and prevent further translation of the septum 114 in a distal direction.

In some embodiments, the needle feature 204 may be configured to catch on a distal end of the septum 114. In some embodiments, withdrawing the introducer needle 112 from the catheter adapter 102 in the proximal direction may urge the needle feature 204 against a distal end of the septum 114. In some embodiments, a withdrawal force exerted on the introducer needle 112 in the proximal direction may in turn cause the needle feature 204 to passively force the septum 114 to slide or otherwise move from the distal position to the proximal position.

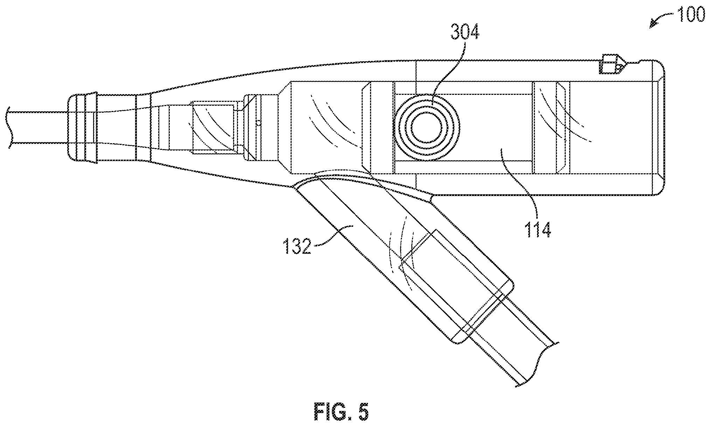

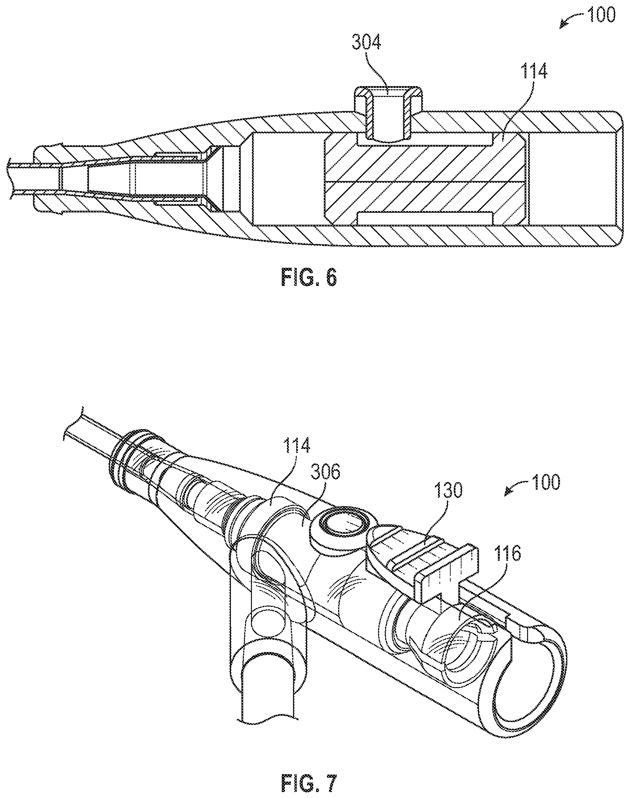

In some embodiments, in the proximal position, as illustrated in Figures 5 and 6, the side port 132 may be in fluid communication with the catheter 110. Further, in some embodiments, the air vent 304 may form a stop to contact the distal edge 308 of the septum 114 within the lumen 108. This may secure the position of the septum 114 with respect to the stop and prevent further translation of the septum 114 in the proximal direction.

In some embodiments, the withdrawal force exerted on the introducer needle 112 in the proximal direction may exceed a force needed to move the septum 114 to the proximal position, causing the needle feature 204 to continue to move proximally through the septum 114. In this manner, the needle feature 204 may be translated through a proximal end of the septum 114, thereby enabling the introducer needle 112 to be removed from the catheter adapter 102.

Referring now to Figures 7 - 10, in some embodiments, the puller element 116 may be coupled to the septum 114 to facilitate translation of the septum 114 between the distal position and the proximal position. In some embodiments, the puller element 116 may be coupled to a proximal end of the septum 114 by a press fit, an interference fit, or another suitable coupling mechanism.

In some embodiments, the puller element 116 may be positioned adjacent to the septum 114 within the lumen 108. In some embodiments, a housing or actuator 800 of a blood collection set 801 may engage a proximal end of the puller element 116 and thereby urge the puller element 116 in a distal direction in response to a force applied in the proximal direction by the clinician. In some embodiments, in response to urging of the puller element 116 in the distal direction, a distal end of the puller element 116 coupled to the proximal end of the septum 114 may automatically slide the septum 114 proximally, such as to the proximal position.

In some embodiments, a proximal end of the puller element 116 may include a male or female connector portion, and a distal end of the actuator 800 may include a male or female connector portion. In some embodiments, a proximal end of the septum 114 may include a male or female connector portion and a distal end of the puller element 116 may include a male or female connector portion. As illustrated, for example, a proximal end of the puller element 116 may include a female connector portion and a distal end of the actuator 800 may include a male connector portion. As also illustrated, for example, a proximal end of the septum 114 may include a female connector portion and a distal end of the puller element 116 may include a male connector portion.

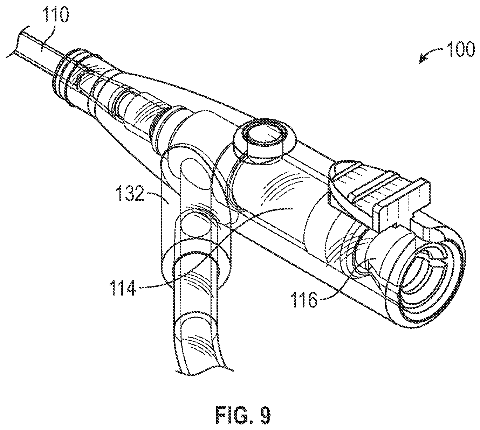

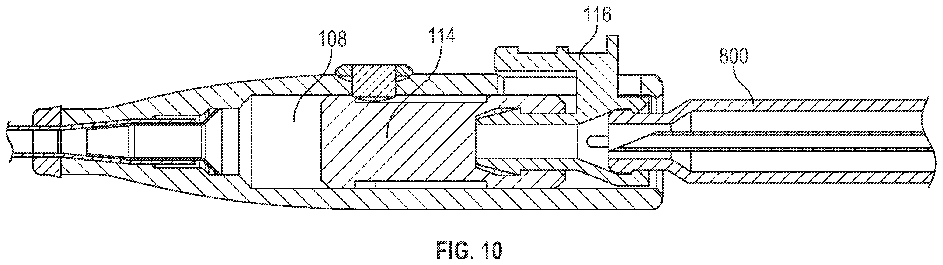

In some embodiments, in operation, the actuator 800 may be inserted into the proximal end 106 of the catheter adapter 102 such that the male connector portion of the actuator 800 engages the female connector portion of the puller element 116. In some embodiments, the actuator 800 may then be urged in the distal direction, causing the male connector portion of the puller element 116 to engage the female connector portion of the septum 114. In some embodiments, continuing to urge the actuator in the distal direction in this manner may cause the septum 114 to slide from the proximal position to the distal position. In some embodiments, as illustrated in Figures 9 and 10, once engaged, the actuator 800 may be moved in the proximal direction. This may cause the septum 114 to passively slide from the distal position to the proximal position, thereby creating a fluid pathway between the side port 132 and the catheter 110.

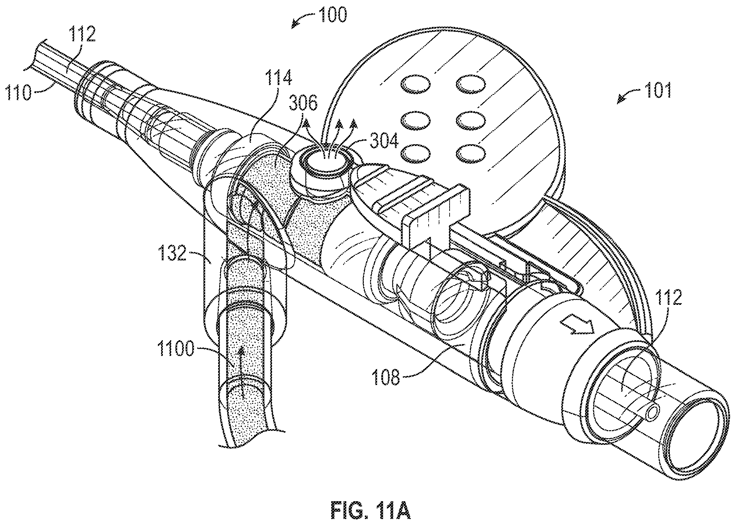

Referring now to Figures 11A-E, a method (not claimed and left for illustrative purposes) may include priming a catheter system 100 with a priming fluid or solution to purge air present out of the catheter system 100 prior to insertion of the catheter 110 into the patient. As illustrated in Figure 11A, priming solution, such as saline or the medication intended for infusion, may be administered through the side port 132 via an extension tube 1100.

In some embodiments, the septum 114 may be initially positioned in the distal position to facilitate a priming process to reduce or eliminate air from the catheter assembly 101. In some embodiments, in the distal position, the septum 114 may occlude the lumen 108 such that priming solution cannot enter or prime the lumen 108 or contact the catheter 110. In some embodiments, preventing exposure of the catheter 110 to priming fluid in this manner may prevent softening of the catheter 110 during insertion. In some embodiments, however, the septum 114 may include an annular groove 306 to permit priming fluid to flow from the side port 132 and across the annular groove 306 to prime a distal portion of the lumen 108 corresponding to the annular groove 306. In some embodiments, the priming fluid may also prime a portion of the air vent 304 exposed to the lumen 108. In some embodiments, the air vent 304 may include a hydrophobic membrane or other suitable air vent to seal the priming fluid within the lumen 108.

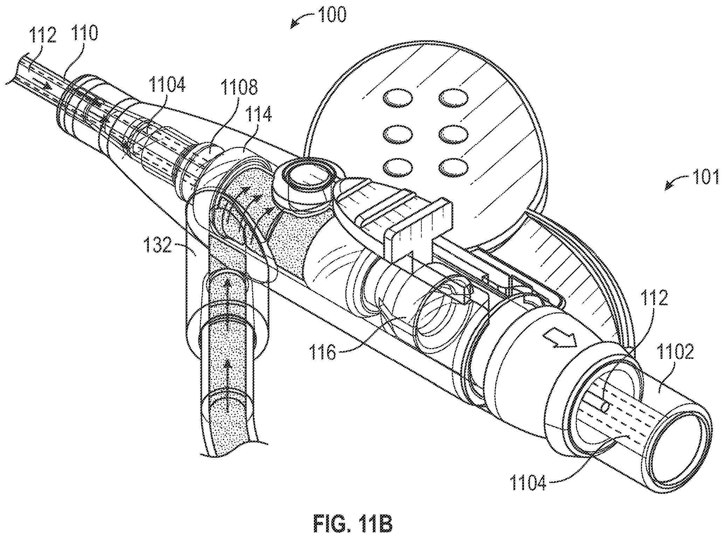

Referring now to Figure 11B, after priming the catheter assembly 101, the introducer needle 112 and catheter 110 may be inserted into a patient's vasculature. In some embodiments, to verify proper placement of the introducer needle 112 and catheter 110 in the blood vessel, the clinician may confirm the presence of "flashback" blood along the catheter 110 and/or in a flashback chamber 1102 disposed within with the catheter assembly 101 and/or needle hub 134. In some embodiments, blood flashback 1104 may flow into the sharp distal tip 202 of the introducer needle 112, through the notch into a space 1108 between the introducer needle 112 and the catheter 110, and proximate the septum 114. Advantageously, in some embodiments, blood may be prevented from fluid communication with the priming solution in response to the septum 114 being in the distal position. In some embodiments, the catheter assembly 101 may facilitate minimal resistance and a quick, clear indication of the blood flashback 1104 within the catheter 110 and/or an indication of blood flashback 1104 at the flashback chamber 1102.

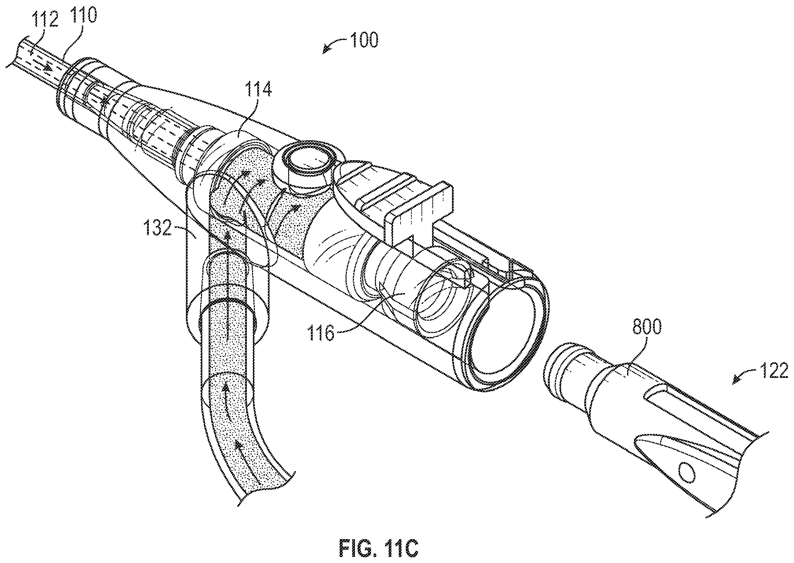

Referring now to Figure 11C, in some embodiments, after inserting the catheter 110 into vein, the clinician may remove the introducer needle 112 from the catheter system 100. In some embodiments, the clinician may then couple the actuator 800 of the blood collection set 801 to the proximal end of the puller element 116 to facilitate continued access to the blood vessel through the catheter for blood collection.

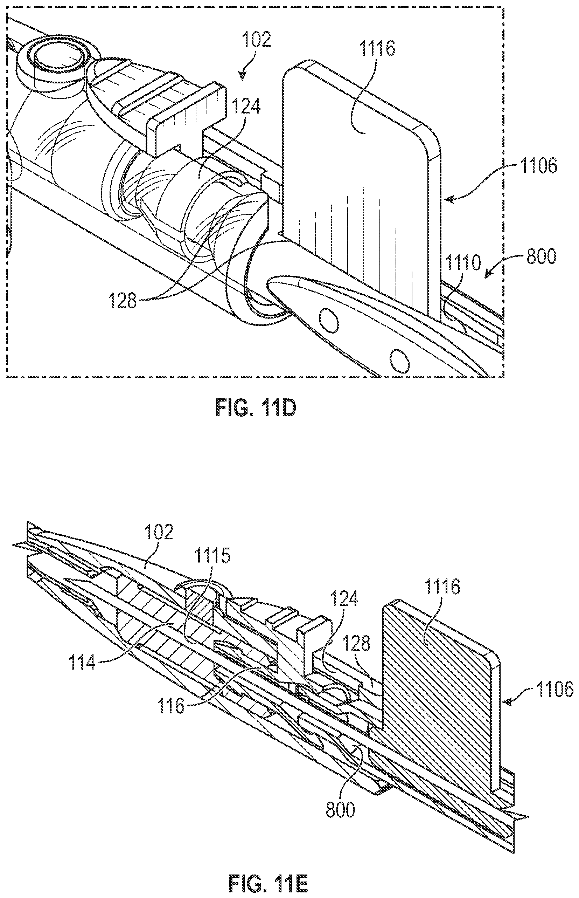

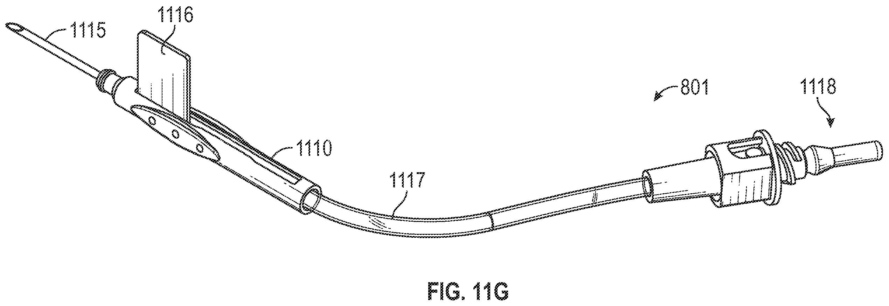

Referring now to Figure 11D and 11G, in some embodiments, a blood collection set 801 may include the actuator 800, which may include a distal end, a proximal end, and a slot 1110. In some embodiments, the blood collection set 801 may include a cannula 1115, which may be coupled to a wing 1116 extending through the slot 1110. In some embodiments, the cannula 1115 may be retained within the actuator when the cannula or needle hub 134 is in a retracted position. In some embodiments, the cannula 1115 may be slidable between the retracted position and an advanced position such that in the advanced position, the cannula 1115 may extend through one or more of the following: the distal end of the actuator 800, the puller element 116, and the septum 114. In some embodiments, a distal end of the cannula 1115 may be disposed within the lumen 108 or the catheter 110. In some embodiments, the wing 1116 may be gripped to facilitate sliding the cannula between the retracted position and the advanced position. In some embodiments, a blood sample may be collected through the blood collection set 801 in response to the cannula 1115 extending through the septum 114.

In some embodiments, the blood collection set 801 may include an extension tube 1117 (see Figure 11G, for example). In some embodiments, the extension tube 1117 may be coupled to the proximal end of the cannula 1115, and a blood collection device 1118 may be coupled to a proximal end of the extension tube 1117. In some embodiments, the blood collection device 1118 may include a syringe, an evacuated blood collection tube, a small sample collection device, or another suitable blood collection device. In some embodiments, the blood collection device 1118 may include a needle enclosed within an elastomeric sheath, as illustrated, for example, in Figure 11G. In some embodiments, the blood collection device 1118 may include a VACUTAINER^{®} available from Becton Dickinson and Company of Franklin Lakes, New Jersey, or another suitable device.

In some embodiments, after collecting the blood sample within the blood collection device 1118 or set 801, the wing 1116 may be gripped and moved along the slot 1110 to slide the cannula 1115 between the advanced position and the retracted position. In some embodiments, the distal end of the cannula 1115 may be disposed within the actuator 800 in response to the cannula 1115 being in the retracted position.

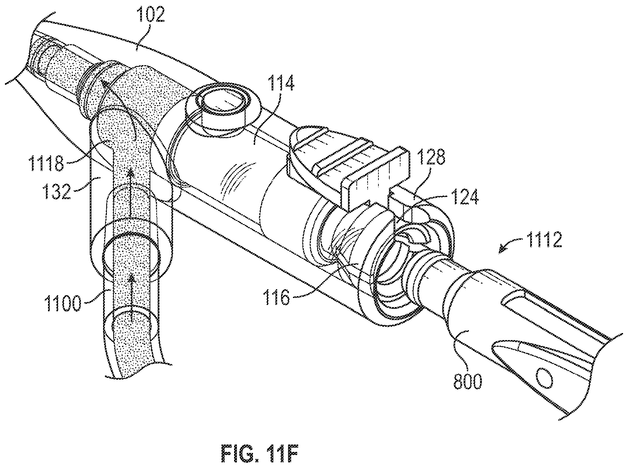

Referring now to Figures 11E and 11F, after collecting a sufficient amount of blood and/or sliding the cannula 1115 to the retracted position, the actuator 800 may be removed from the puller element 116. In some embodiments, the actuator 800 may be removed by applying force in the proximal direction such that the puller element 116 contacts the stop 128 of the catheter adapter 102 slot 124. Continued force on the actuator 800 in a proximal direction may cause the actuator 800 to disengage from the puller element 116. Additionally, referring now to Figure 11E, in some embodiments, proximal movement of the actuator 800 in this manner may passively slide the septum 114 from the distal position to the proximal position, thereby opening a fluid path that includes the catheter 110, a portion of the lumen 108, the side port 132, and extension tube 1100, as illustrated in Figure 11F.

All examples and conditional language recited herein are intended for pedagogical objects to aid the reader in understanding the invention and the concepts contributed by the inventor to furthering the art, and are to be construed as being without limitation to such specifically recited examples and conditions. Although embodiments of the present inventions have been described in detail, it should be understood that the various changes, substitutions, and alterations could be made hereto without departing from the scope of the invention.

## Claims

1. A catheter assembly (101), comprising:
a catheter adapter (102), comprising a distal end (104), a proximal end (106), a lumen (108) extending through the distal end (104) and the proximal end, and a side port (132) between the distal end (104) of the catheter adapter (102) and the proximal end of the catheter adapter (102);
a catheter (110) extending from the distal end (104) of the catheter adapter (102);
a septum (114) disposed within the lumen (108) at a distal position aligned with the side port (132), wherein in response to the septum being in the distal position, the septum provides a seal between the side port (132) and the catheter (110),
**characterized in that**
the catheter assembly (101) comprises a puller element (116) disposed within the lumen (108), and the catheter adapter (102) comprises a slot (124) having a proximal end that comprises a stop (128), wherein the puller element (116) comprises a distal end (118) coupled to the septum, a proximal end (120) configured to couple to a blood collection set (801), and an extension (26) extending through the slot (124) and configured to contact the stop (128) in response to the septum sliding from the distal position to the proximal position,
wherein the septum is configured to slide from the distal position to a proximal position proximal to the side port (132), wherein in response to the septum being in the proximal position, the side port (132) is in fluid communication with the catheter (110).

2. The catheter assembly (101) of claim 1, wherein the extension is coupled to a push tab (130).

3. The catheter assembly (101) of claim 1, wherein the catheter adapter (102) comprises an air vent (304), wherein an outer surface of the septum comprises an annular groove (306), wherein in response to the septum being in the distal position, the side port (132) is in fluid communication with the annular groove and the air vent (304).

4. A catheter system (100), comprising:
a catheter assembly (101), comprising:
a catheter adapter (102), comprising a distal end (104), a proximal end (106), a lumen (108) extending through the distal end (104) and the proximal end, and a side port (132) between the distal end (104) of the catheter adapter (102) and the proximal end of the catheter adapter (102);
a catheter (110) extending from the distal end (104) of the catheter adapter (102);
a stop (128) within the lumen (108); and
a septum (114) disposed within the lumen (108) at a distal position aligned with the side port (132), wherein in response to the septum (114) being in the distal position, the septum (114) provides a seal between the side port (132) and the catheter (110); and
a needle assembly (200), comprising:
a needle hub (134) coupled to the proximal end (106) of the catheter adapter (102);
an introducer needle (112), comprising a sharp distal tip (202), a proximal end (106) (206), and a needle feature (204) disposed between the sharp distal tip and the proximal end (106), wherein the proximal end (106) of the introducer needle (112) is secured within the needle hub (134),
**characterized in that**
wherein in response to withdrawal of the introducer needle (112) in a proximal direction a first amount, the needle feature is configured to contact the septum (114) and slide the septum from the distal position to a proximal position, wherein in response to the septum (114) being in the proximal position, the side port (132) is in fluid communication with the catheter (110) and the stop (128) contacts the septum (114), wherein in response to withdrawal of the introducer needle (112) in the proximal direction more than the first amount, the needle feature is configured to move proximally with respect to the septum (114).

5. The catheter system (100) of claim 4, wherein the needle feature comprises a bump.

6. The catheter system of claim 4, wherein the catheter adapter (102) comprises an air vent (304), wherein an outer surface of the septum (114) comprises an annular groove (306), wherein in response to the septum (114) being in the distal position, the side port (132) is in fluid communication with the annular groove and the air vent (304).

7. The catheter system (100) of claim 6, wherein the air vent (304) forms the stop (128).

## Patentansprüche

1. Katheterbaugruppe (101), die aufweist:
einen Katheteradapter (102), der ein distales Ende (104), ein proximales Ende (106), ein Lumen (108), das sich durch das distale Ende (104) und das proximale Ende erstreckt, und eine Seitenport (132) zwischen dem distalen Ende (104) des Katheteradapters (102) und dem proximalen Ende des Katheteradapters (102) aufweist;
einen Katheter (110), der sich ausgehend von dem distalen Ende (104) des Katheteradapters (102) erstreckt;
ein Septum (114), das innerhalb des Lumens (108) an einer distalen Position angeordnet ist, die mit dem Seitenport (132) ausgerichtet ist, wobei als Reaktion darauf, dass sich das Septum in der distalen Position befindet, das Septum eine Dichtung zwischen dem Seitenport (132) und dem Katheter (110) bildet,
**dadurch gekennzeichnet, dass**
die Katheterbaugruppe (101) ein Ziehelement (116) aufweist, das innerhalb des Lumens (108) angeordnet ist, und der Katheteradapter (102) einen Schlitz (124) mit einem proximalen Ende aufweist, das einen Anschlag (128) aufweist, wobei das Ziehelement (116) ein distales Ende (118), das mit dem Septum gekoppelt ist, ein proximales Ende (120), das so ausgebildet ist, dass es mit einem Blutentnahmeset (801) gekoppelt wird, und eine Verlängerung (26) aufweist, die sich durch den Schlitz (124) erstreckt und so ausgebildet ist, dass sie den Anschlag (128) als Reaktion auf das Gleiten des Septums von der distalen Position in die proximale Position berührt,
wobei das Septum so ausgebildet ist, dass es von der distalen Position in eine proximale Position proximal zum Seitenport (132) gleitet, wobei als Reaktion darauf, dass sich das Septum in der proximalen Position befindet, der Seitenport (132) in Fluidverbindung mit dem Katheter (110) steht.

2. Katheterbaugruppe (101) nach Anspruch 1, wobei die Verlängerung mit einer Drücklasche (130) gekoppelt ist.

3. Katheterbaugruppe (101) nach Anspruch 1, wobei der Katheteradapter (102) eine Entlüftungsöffnung (304) aufweist, wobei eine Außenfläche des Septums eine ringförmige Nut (306) aufweist, wobei als Reaktion darauf, dass sich das Septum in der distalen Position befindet, der Seitenport (132) in Fluidverbindung mit der ringförmigen Nut und der Entlüftungsöffnung (304) steht.

4. Kathetersystem (100), das aufweist:
eine Katheterbaugruppe (101), die aufweist:
einen Katheteradapter (102), der ein distales Ende (104), ein proximales Ende (106), ein Lumen (108), das sich durch das distale Ende (104) und das proximale Ende erstreckt, und eine Seitenport (132) zwischen dem distalen Ende (104) des Katheteradapters (102) und dem proximalen Ende des Katheteradapters (102) aufweist;
einen Katheter (110), der sich ausgehend von dem distalen Ende (104) des Katheteradapters (102) erstreckt;
einen Anschlag (128) innerhalb des Lumens (108); und
ein Septum (114), das innerhalb des Lumens (108) an einer distalen Position angeordnet ist, die mit dem Seitenport (132) ausgerichtet ist, wobei als Reaktion darauf, dass sich das Septum (114) in der distalen Position befindet, das Septum (114) eine Dichtung zwischen dem Seitenport (132) und dem Katheter (110) bildet; und
eine Nadelbaugruppe (200), die aufweist:
ein Nadelansatz (134), der mit dem proximalen Ende (106) des Katheteradapters (102) verbunden ist;
eine Einführnadel (112), die eine scharfe distale Spitze (202), ein proximales Ende (106) (206) und ein zwischen der scharfen distalen Spitze und dem proximalen Ende (106) angeordnetes Nadelmerkmal (204) aufweist, wobei das proximale Ende (106) der Einführnadel (112) innerhalb des Nadelansatzes (134) befestigt ist,
**dadurch gekennzeichnet, dass**
wobei als Reaktion auf das Zurückziehen der Einführnadel (112) in einer proximalen Richtung um ein erstes Maß das Nadelmerkmal so ausgebildet ist, dass es das Septum (114) berührt und das Septum von der distalen Position in eine proximale Position verschiebt, wobei als Reaktion darauf, dass das Septum (114) in der proximalen Position ist, der Seitenport (132) in Fluidverbindung mit dem Katheter (110) steht und der Anschlag (128) das Septum (114) berührt, wobei als Reaktion auf das Zurückziehen der Einführnadel (112) in der proximalen Richtung um mehr als das erste Maß das Nadelmerkmal so ausgebildet ist, dass es sich in Bezug auf das Septum (114) proximal bewegt.

5. Kathetersystem (100) nach Anspruch 4, wobei das Nadelmerkmal eine Erhebung aufweist.

6. Kathetersystem nach Anspruch 4, wobei der Katheteradapter (102) eine Entlüftungsöffnung (304) aufweist, wobei eine Außenfläche des Septums (114) eine ringförmige Nut (306) aufweist, wobei als Reaktion darauf, dass sich das Septum (114) in der distalen Position befindet, der Seitenport (132) in Fluidverbindung mit der ringförmigen Nut und der Entlüftungsöffnung (304) steht.

7. Kathetersystem (100) nach Anspruch 6, wobei die Entlüftungsöffnung (304) den Anschlag (128) bildet.

## Revendications

1. Ensemble cathéter (101), comprenant
un adaptateur de cathéter (102), comprenant une extrémité distale (104), une extrémité proximale (106), une lumière (108) s'étendant à travers l'extrémité distale (104) et l'extrémité proximale, et un orifice latéral (132) entre l'extrémité distale (104) de l'adaptateur de cathéter (102) et l'extrémité proximale de l'adaptateur de cathéter (102) ;
un cathéter (110) s'étendant à partir de l'extrémité distale (104) de l'adaptateur de cathéter (102) ;
un septum (114) disposé à l'intérieur de la lumière (108), au niveau d'une position distale alignée avec l'orifice latéral (132), où, en réponse au fait que le septum se trouve dans la position distale, le septum fournit un joint d'étanchéité entre l'orifice latéral (132) et le cathéter (110),
**caractérisé en ce que**
l'ensemble cathéter (101) comprend un élément de traction (116) disposé à l'intérieur de la lumière (108), et **en ce que** l'adaptateur de cathéter (102) comprend une fente (124) présentant une extrémité proximale qui comprend une butée (128), où l'élément de traction (116) comprend une extrémité distale (118) couplée au septum, une extrémité proximale (120) configurée pour se coupler à un ensemble de collecte de sang (801), et une extension (26) s'étendant à travers la fente (124) et configurée pour venir en contact avec la butée (128) en réponse au fait que le septum coulisse depuis la position distale vers la position proximale,
dans lequel le septum est configuré pour coulisser depuis la position distale vers une position proximale par rapport à l'orifice latéral (132), où, en réponse au fait que le septum se trouve dans la position proximale, l'orifice latéral (132) se trouve en communication fluidique avec le cathéter (110).

2. Ensemble cathéter (101) de la revendication 1, dans lequel l'extension est couplée à une languette de poussée (130).

3. Ensemble cathéter (101) de la revendication 1, dans lequel l'adaptateur de cathéter (102) comprend un évent (304), dans lequel une surface extérieure du septum comprend une rainure annulaire (306), dans lequel, en réponse au fait que le septum se trouve dans la position distale, l'orifice latéral (132) se trouve en communication fluidique avec la rainure annulaire et l'évent (304).

4. Système de cathéter (100), comprenant :
un ensemble cathéter (101), comprenant :
un adaptateur de cathéter (102), comprenant une extrémité distale (104), une extrémité proximale (106), une lumière (108) s'étendant à travers l'extrémité distale (104) et l'extrémité proximale, et un orifice latéral (132) entre l'extrémité distale (104) de l'adaptateur de cathéter (102) et l'extrémité proximale de l'adaptateur de cathéter (102) ;
un cathéter (110) s'étendant depuis l'extrémité distale (104) de l'adaptateur de cathéter (102) ;
une butée (128) à l'intérieur de la lumière (108) ; et
un septum (114) disposé à l'intérieur de la lumière (108) au niveau d'une position distale alignée avec l'orifice latéral (132), où, en réponse au fait que le septum (114) se trouve dans la position distale, le septum (114) fournit un joint d'étanchéité entre l'orifice latéral (132) et le cathéter (110) ; et
un ensemble d'aiguille (200), comprenant :
une embase (134) couplée à l'extrémité proximale (106) de l'adaptateur de cathéter (102) ;
une aiguille d'introduction (112), comprenant une pointe distale pointue (202), une extrémité proximale (106) (206), et un élément d'aiguille (204) disposé entre la pointe distale pointue et l'extrémité proximale (106), où l'extrémité proximale (106) de l'aiguille d'introduction (112) est fixée à l'intérieur de l'embase (134),
**caractérisé en ce que**
en réponse à un retrait de l'aiguille d'introduction (112) dans une direction proximale d'une première quantité, l'élément d'aiguille est configuré pour venir en contact avec le septum (114) et faire coulisser le septum depuis la position distale vers une position proximale, où, en réponse au fait que le septum (114) de trouve dans la position proximale, l'orifice latéral (132) se trouve en communication fluidique avec le cathéter (110) et la butée (128) vient en contact avec le septum (114), où, en réponse à un retrait de l'aiguille d'introduction (112) dans la direction proximale supérieur à la première quantité, l'élément d'aiguille est configuré pour se déplacer de manière proximale par rapport au septum (114).

5. Système de cathéter (100) de la revendication 4, dans lequel l'élément d'aiguille comprend un bossage.

6. Système de cathéter de la revendication 4, dans lequel l'adaptateur de cathéter (102) comprend un évent (304), où une surface extérieure du septum (114) comprend une rainure annulaire (306), dans lequel, en réponse au fait que le septum (114) se trouve dans la position distale, l'orifice latéral (132) se trouve en communication fluidique avec la rainure annulaire et l'évent (304).

7. Système de cathéter (100) de la revendication 6, dans lequel l'évent (304) forme la butée (128).
